# EUROPEAN PATENT SPECIFICATION

(11) **EP 3 945 315 B1**
(45) Date of publication and mention of the grant of the patent: **07.10.2026**
(21) Application number: 21188942.3
(22) Date of filing: 30.07.2021
(51) Int. Cl.: G01N 33/00

(54) **GAS DETECTOR DEVICE**
GAS DETECTOR DEVICE
DISPOSITIF DE DÉTECTION DE GAZ

(30) Priority: 30.07.2020 IT 202000018679
(43) Date of publication of application: 02.02.2022
(73) Proprietor: Taua S.r.l., 40129 Bologna (IT)
(72) Inventor: LEPRI, Davide, 40122 Bologna (IT); BELLOMO, Marco, 40050 Castello d'Argile (BO) (IT); ROMAGNOLI, Andrea, 40129 Bologna (IT)
(74) Representative: Studio Torta S.p.A.

(56) References cited:
- US-A- 6 085 576
- US-A1- 2006 188 399
- US-A1- 2018 202 984
- US-A1- 2018 353 885

## Description

### CROSS-REFERENCE TO RELATED APPLICATIONS

This patent application claims priority from Italian patent application no. 102020000018679 filed on 30/07/2020.

### TECHNICAL FIELD

The present invention relates to a gas detector device.

### TECHNICAL BACKGROUND

Gas detector devices comprising a protective casing and an electronic circuit housed in the casing, provided with a gas sensor and designed to detect a gas flowing inside the casing through gas supply openings are known.

The document EP2102630 describes a sensor for monitoring the presence of gas in hermetic packages for electronic devices that contain elements that are sensitive to contact with atmospheric gases. The electronic device is encapsulated in a shell inside which also the sensor is present that detects the entry into the shell of gases such as oxygen and humidity. The sensor is of the electrically conductive type. The document US5621162 describes a gas sensor for food packaging. The sensor is mounted by means of platinum wires to at least two pins. It is then enclosed inside a casing formed by a resin base and a metal cap provided with openings to allow the inlet of gas.

The document WO2016/163630 describes a thin-film sensor for detecting oxygen and carbon dioxide in a package in real time. It is not provided with an outer shell.

The document WO03044521 describes a sensor, e.g. for detecting oxygen, which is readable by RF techniques and which is placed inside a hermetic package of a foodstuff. This sensor is not provided with an outer shell.

The document US7811433 describes an electrochemical sensor for detecting carbon dioxide also inside a food package. The sensor is placed inside a plastic casing (housing 20). The casing is provided with at least one opening through which the gas enters to reach the sensor.

Such detector devices are designed to detect a specific type of gas (or a group of specific gases) and are thus not of a reconfigurable type should the need arise to detect different gases.

Document US2018/202984 disclosing a water-resistant enclosure provided with a base and a lid which can be coupled to the base. The enclosure houses a controller, a power source and a gas sensor which is arranged on the lid. A gasket internally separates the cover of the base. A permeable membrane in gas-permeable silicone covers the sensor on an upper side while on the lower side the sensor is held by a layer of polymer (for example a polyurethane glue). In particular, the polymer holds the body of the sensor stably.

The object of the present invention is to provide a gas detector device which allows an easy replacement of the gas sensor while keeping the same control electronics. A further object of the present invention is to provide a gas sensor having a simple, plain and robust construction.

### SUMMARY OF THE INVENTION

The preceding object is achieved by the present invention since the latter relates to a gas detector device of the type described in claim 1.

### BRIEF DESCRIPTION OF THE DRAWINGS

The invention will now be illustrated with reference to the accompanying drawings, which represent a preferred nonlimiting embodiment thereof, wherein:
Figure 1 illustrates a perspective view of a gas detector device manufactured according to the principles of the present invention;
Figure 2 illustrates a perspective view partially in section of a gas detector device manufactured according to the principles of the present invention;
Figure 3 illustrates a perspective view partially in section with parts removed of a gas detector device manufactured according to the principles of the present invention;
Figure 4 illustrates an exploded view of a gas detector device manufactured according to the principles of the present invention;
Figure 5 illustrates the operations performed by a microprocessor of the gas detector device according to the present invention; and
Figure 6 illustrates a variant, not part of the present invention, of the device of Figures 1-5.

### DESCRIPTION OF THE EXAMPLE EMBODIMENT

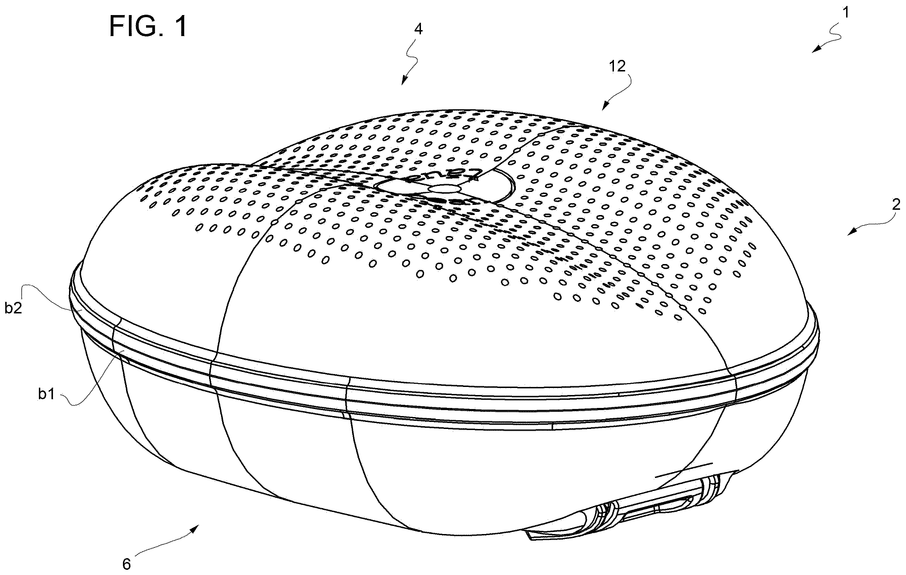

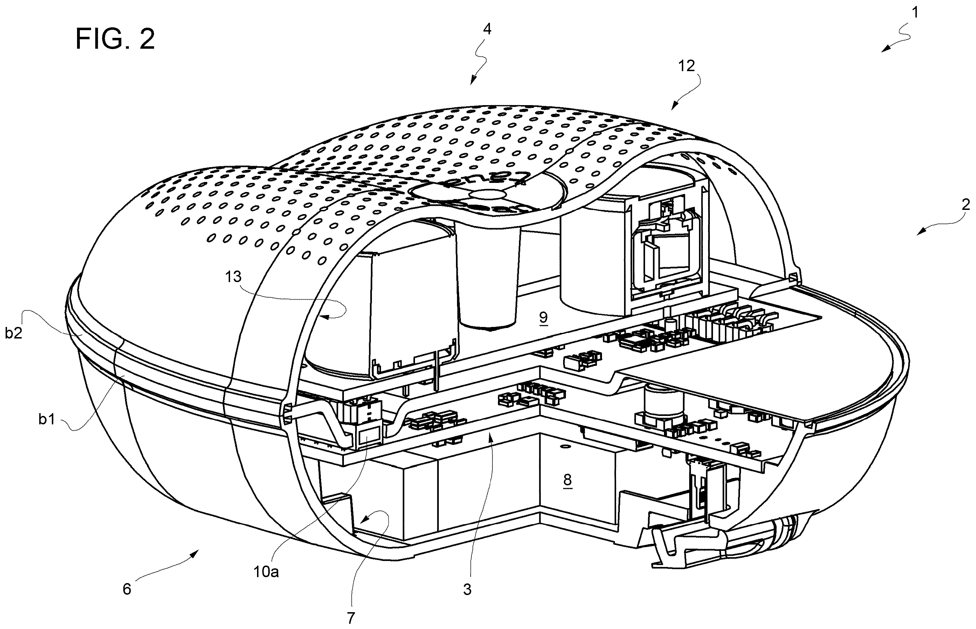

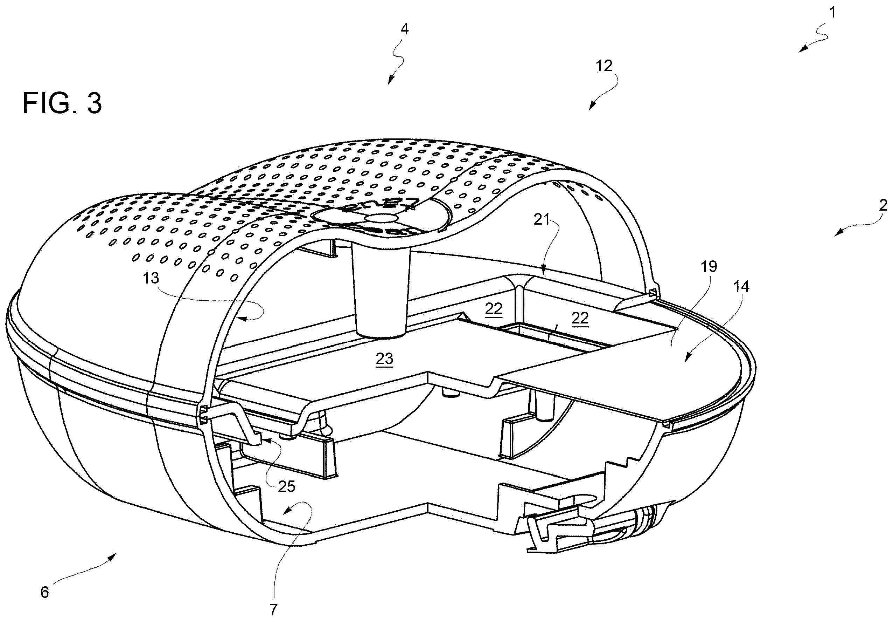

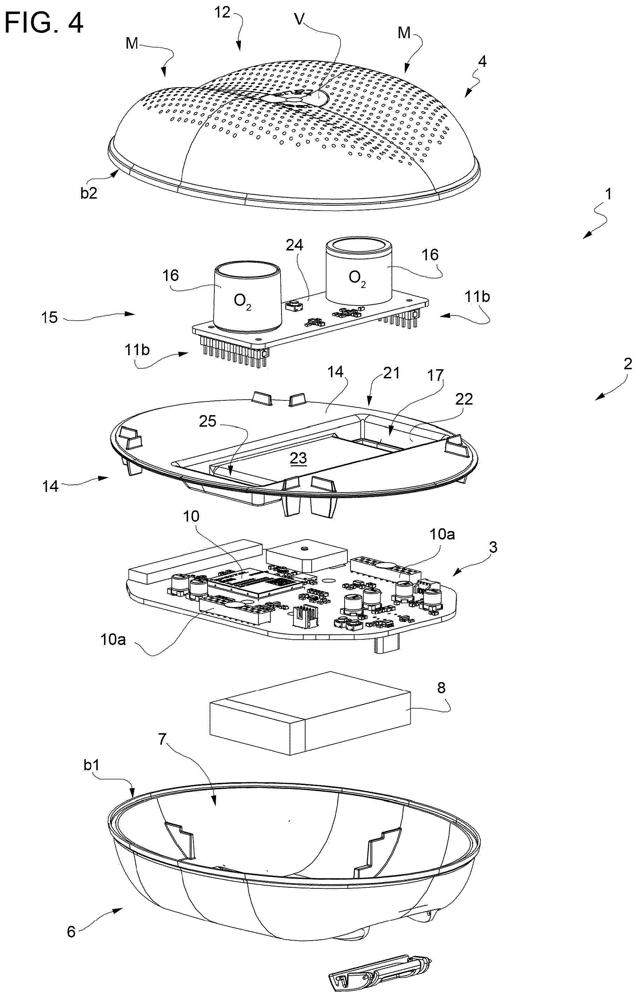

With reference to Figures 1 to 4, reference numeral 1 indicates, as a whole, a gas detector device comprising a protective casing 2 and an electronic circuit 3 (of a known type) housed in the casing 2 and designed to detect a gas flowing inside the casing 2 through gas supply openings 4 made in a wall of said casing 2.

The electronic circuit 3 is designed to detect, by means of known technologies, the presence and potentially the concentration of a type of gas (or more types of different gases) and is designed to communicate with a control remote station with the modalities described in the following. Among the detectable gases we hereby mention, by way of nonlimiting example, oxygen, carbon dioxide, nitrogen, aromatic compounds, hydrocarbons and CO₂ for food processing.

According to the present invention, the casing 2 comprises:
a first half-shell 6 defining a first internal seat 7 configured to house a power-supply battery 8 (of a known type, for example a lithium-ion battery) and a main board 9 carrying the control electronics of the gas detector device including a microprocessor 10 and a series of first electrical connectors 10a (in the example, a pair of linearly arranged connectors, the shape and the number of which can be different):
a second half-shell 12 defining a second internal seat 13, configured to couple with the first half-shell 6 in a sealed manner and provided with a plurality of through-openings 4 configured to establish communication between the outside of the casing 2 and the second internal seat 13; and
a partition septum 14 designed to be housed inside the casing 2 to separate the first internal seat 7 from the second internal seat 13 by arranging it facing the first connectors 10a of the main board 9.

The first half-shell can contain the power-supply battery 8 and the main board 9 in an impermeable manner, for example by arranging the battery and the board 9 inside a fluid material (of a known type), which solidifies and fills the first half-shell while enveloping such components. The partition septum 14 is provided with a seat 17 configured to support a removable base 15 provided with at least one sensor 16 (two sensors 16 with a cylindrical structure are illustrated in the example) for a specific type of gas and provided with second electrical connectors 11b (a pair of connectors in the example, the shape and the number of which can be different) that couple in a releasable manner with the first electrical connectors 10a when the removable base 15 is arranged in the seat 17.

The partition septum 14 comprises a flat wall 19 provided with a rectangular lowered portion 21 that creates the seat 17 and is limited by side walls 22 and a back wall 23. The removable base 15 comprises a supporting portion 24 (made of a rectangular portion of printed circuit in the example) that is shaped to be housed in the lowered portion 21 and supports the sensor 16.
The lowered portion 21 is provided with two through-openings 25 which open in proximity to end portions of the back wall 23 and which are designed to allow the passage of the second electrical connectors 11b in order to achieve the coupling with the first connectors 10a of the main board 9. In the example, a pair of rectangular through-openings 25 are provided so as to create the passage of the respective connectors 11b, but the number and the shape can obviously be different depending on the connectors used.

The first half-shell 6 is cup-shaped and has a substantially C-shaped cross-section, while the second half-shell 12 is cup-shaped and has a substantially omega-shaped cross-section so that it has, when viewed in cross-section, a lowered central portion V compared to raised lateral portions M. The flat wall 19 has a substantially elliptical shape in plan.
The openings 4 are made in the lowered central portion V and in the lateral portions M and are arranged according to a matrix ordered structure.
The first half-shell 6 and the second half-shell 12 are made of an impact-resistant plastic material, such as ABS.

**In use,** in order to carry out the replacement of the sensor 16, an operator manually uncouples the first half-shell 6 from the second half-shell 12. For example, the half-shells 6, 12 can be provided with a snap-fitting coupling device (of a known type) interposed between the free edges b1, b2 facing each other of the two half-shells 6 and 12. The coupling device can be of different nature, e.g., made by means of screws (not illustrated) or glued.
The removal of the second half-shell 12 allows having access to the base 15, which can be removed by uncoupling the first and the second connectors 10a, 11b. In this manner, a base 15 carrying a specific sensor 16 (e.g. a carbon dioxide sensor) is removed. A new base 15 is then mounted, which carries a different sensor 16 (e.g. an oxygen sensor). By arranging the base 15 in the seat 17, the first and second electrical connectors 10a,11b are coupled.

The presence of a seat 17 facilitates the arrangement of the base 15 in the correct position in which the first electrical connectors 11a couple with the second electrical connectors 11b, facilitating the replacement operations of the sensor 16.
The operations end with the arrangement of the half shell 12 on top of the half shell 6.
The sensor device 1 can be arranged in any environment for the gas detection. According to a specific application, the sensor device 1 can be arranged in a bag-shaped container containing food products. The particular shape of the second half-shell 12 (omega-shaped cross-section) prevents the bag from being arranged in such a manner to entirely cover the openings **4,** thereby preventing the inlet of the gas into the second seat 13.

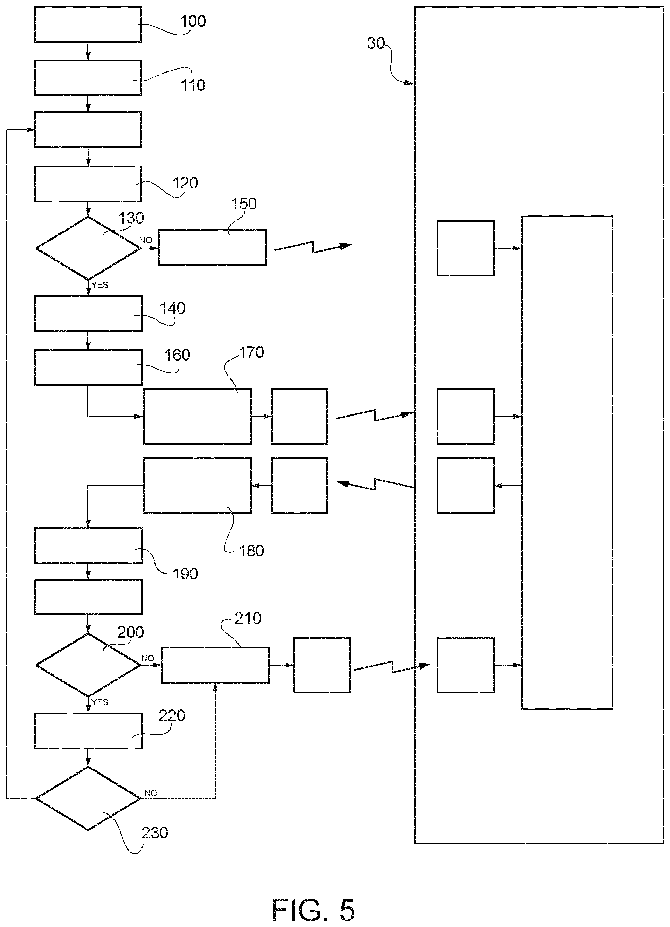

The operations performed by the microprocessor 10 are illustrated with reference to Figure 5.

The microprocessor 10 is configured to perform the following operations:
start a wake-up cycle (Wake UP) of the microprocessor itself (block 100) starting from the very moment of awakening T_{WA};
when the microprocessor 10 wakes up, start a control cycle of the remaining charge **Qb** present in the battery 8 (block 110);
calculate the expected amount of charge Qm required to perform a work cycle (task) of the sensor device 1 (block 120);
check (block 130) whether the remaining charge **Qb** is equal to or greater than the expected amount of charge Qm and whether the work cycle (task) is achievable (sufficient autonomy of the battery 8);
in the case of a positive outcome from block 130, the main board 9 is powered in order to perform the detection of gas through the sensor 16 (block 140).
in the case of a negative outcome from block 130, a signal of a low battery state is transmitted (block 150) using known technologies such as GSM or Wireless. The signal is transmitted to a control remote station 30 (shown schematically) where the data are stored in a safe and unmodifiable form, e.g. using the blockchain technology.

The block 140 is followed by a block 160 which detects the position of the sensor 1 by means of a GPS unit of a known type provided on the main board 9.

The block 160 is followed by a block 170 which transmits the following measurement data to the remote station 30:
percentage of gas detected by the sensor 16;
detected position;
remaining charge **Qb.**

The data are also saved on a fixed medium (flash memory, for example).

After the correct transmission and reception of data, a block 180 detects control data coming from the remote station 30 for the setting of the microprocessor 10.

Such control data are designed to determine a subsequent moment of awakening of the microprocessor 10.

The block 190 is followed by a block 200 which detects the voltage **V_{b}** of the battery 8; if the measured voltage **V_{b}** is lower than a threshold **Vₜₕ**, an insufficient voltage (**V_{b}** < **Vₜₕ**) is detected and a signal of a low battery state is transmitted (block 210) using known technologies such as GSM or Wireless. The signal is transmitted to the control remote station 30.

If, instead, the battery is sufficiently charged, the block 200 is followed by a block 220 which performs the following operations:
- the voltage of the battery is measured again (block 230);
- if the measured voltage is lower than a first threshold V1, the transmission of a completely uncharged battery status is performed using known technologies such as GSM or Wireless.
- if the voltage is lower than a second threshold V2 (higher than the first threshold V1), an estimation of the future consumption is performed based on a series of foreseen operations and it is checked whether the measured voltage is sufficient for the battery to perform these operations. Also in this case, the information on the state is transmitted (low battery but able to perform a number of operations).

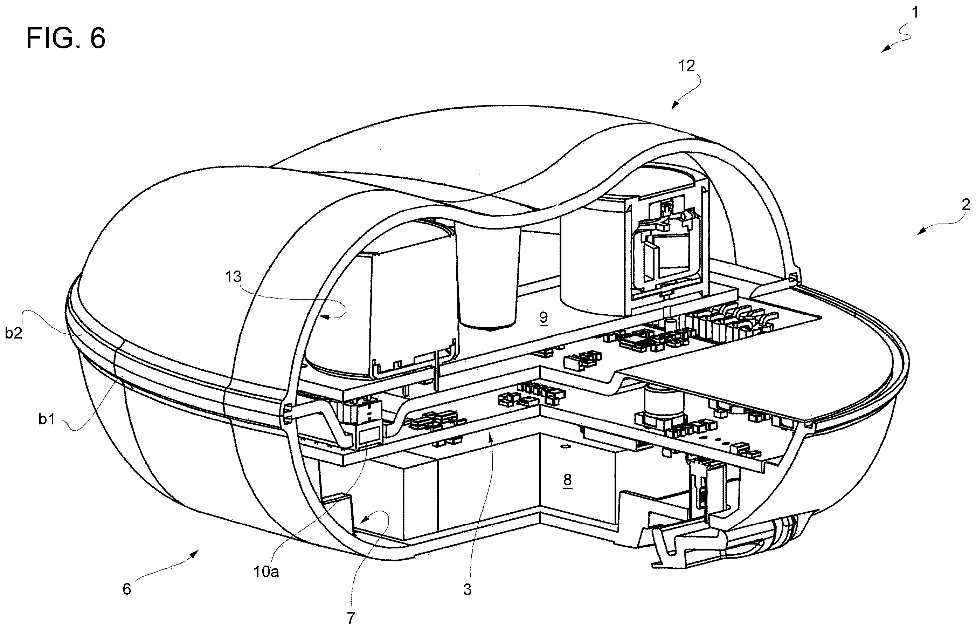

In Figure 6 a variant, not part of the present invention, to the detector device described in the foregoing is illustrated. The mechanical structure is the same, whereas the sensor is a sensor that detects a generic quantity such as an acceleration, a temperature, etc.

The through-openings 4 can be not present (as shown in Figure 6) or can be present.
A quantity detector device 1 comprising a protective casing 2 and an electronic circuit 9 housed in the casing 2 and designed to detect and measure such quantity is thus manufactured.

The casing comprises:
a first half-shell 6 defining a first internal seat 7 configured to house a power-supply battery 8 and a main board 9 carrying the control electronics of the detector device including the microprocessor 10 and a series of first electrical connectors 10a:
a second half-shell 12 defining a second internal seat 13, configured to couple with the first half-shell 6;
a partition septum 14 designed to be housed inside the casing 2 to separate the first internal seat 7 from the second internal seat 13 by arranging it facing the first connectors 11a of the main board:
   the partition septum 14 is provided with a seat 17 configured to house a removable base 15 provided with at least one sensor 16 for a specific quantity and provided with second electrical connectors 11b that couple in a releasable manner with the first electrical connectors 10a when the base 15 is arranged in the seat 17.

## Claims

1. A gas detector device (1) comprising a protective casing (2), at least one sensor (16) for a specific type of gas housed in the protective casing (2) and an electronic circuit (9) housed in the casing (2) and designed to detect a gas flowing inside the casing (2) through gas supply openings (4), said casing comprising:
i) a first half-shell (6) defining a first internal seat (7);
ii) a second half-shell (12) defining a second internal seat (13), configured to couple with the first half-shell (6) and provided with the plurality of through-openings (4) that are configured to establish communication between the outside of the casing (2) and the second internal seat (13); and
iii) a partition septum (14),
**characterised in that** said gas detector further comprises:
a power-supply battery (8) housed in said first internal seat (7);
a main board (9) carrying the control electronics of the detector device including the microprocessor (10) and a series of first electrical connectors (10a) housed in said first internal seat (7);
the partition septum (14) is housed inside said casing (2) to separate the first internal seat (7) from the second internal seat (13) by arranging it facing the first connectors (10a) of the main board:
a removable base (15) provided with at least one sensor (16); said partition septum (14) being provided with a seat (17) configured to house the removable base (15) that is provided with second electrical connectors (11b) that couple in a releasable manner with the first electrical connectors (10a) when the base (15) is arranged in the seat (17).

2. The gas detector device according to claim **1,** wherein the partition septum (14) comprises a flat wall (19) provided with a lowered portion (21) that creates the seat (17) and is limited by side walls (22) and a back wall (23); the removable base (15) comprises a supporting portion (24) that is shaped to be housed in the lowered portion (21) and supports the sensor (16).

3. The gas detector device according to claim 2, wherein the lowered portion (21) is provided with through-openings (25) designed to allow the passage of the first or second electrical connectors (10a, 11b) in order to achieve the coupling between the connectors (10a, 11b).

4. The gas detector device according to any one of the preceding claims, wherein the first half-shell (6) is cup-shaped and has a substantially C-shaped cross-section while the second half-shell is cup-shaped and has a substantially omega-shaped cross-section so that it has, when seen from the outside, a lowered central portion (V) compared to raised lateral portions (M).

5. The gas detector device according to claim **4,** wherein the openings (4) are made in the lowered central portion and in the lateral portions and are arranged in a matrix ordered structure.

6. The gas detector device according to any one of the preceding claims, wherein snap-fitting means are interposed between the free edges (b1,b2) of the first cup-shaped half-shell (6) and the second cup-shaped half-shell (12).

7. The gas detector device according to any one of the preceding claims, wherein connection means are interposed between the free edges (b1,b2) of the first cup-shaped half-shell (6) and second cup-shaped half-shell (12), connection means comprising screws and/or an adhesive.

## Patentansprüche

1. Gasdetektorvorrichtung (1), umfassend ein Schutzgehäuse (2), mindestens einen Sensor (16) für eine bestimmte Gasart, der in dem Schutzgehäuse (2) untergebracht ist, und eine elektronische Schaltung (9), die in dem Gehäuse (2) untergebracht und ausgestaltet ist, um ein Gas zu detektieren, das durch Gaszufuhröffnungen (4) in das Innere des Gehäuses (2) strömt, wobei das Gehäuse Folgendes umfasst:
i) eine erste Halbschale (6), die eine erste interne Aufnahme (7) definiert;
ii) eine zweite Halbschale (12), die eine zweite interne Aufnahme (13) definiert, ausgelegt ist, um mit der ersten Halbschale (6) gekuppelt zu werden und mit der Vielzahl von Durchgangsöffnungen (4) versehen ist, die ausgelegt sind, um eine Kommunikation zwischen der Außenseite des Gehäuses (2) und der zweiten internen Aufnahme (13) herzustellen, und
iii) eine Teilungswand (14),
**dadurch gekennzeichnet, dass** der Gasdetektor ferner folgendes umfasst: eine Stromversorgungsbatterie (8), die in der ersten internen Aufnahme (7) untergebracht ist;
eine Hauptplatine (9), die die Steuerelektronik der Detektorvorrichtung trägt, den Mikroprozessor (10) sowie eine Reihe von ersten elektrischen Verbindern (10a) beinhaltet und in der ersten internen Aufnahme (7) untergebracht ist,
wobei die Teilungswand (14) im Inneren des Gehäuses (2) untergebracht ist, um durch ihre den ersten Verbindern (10a) der Hauptplatine zugewandte Anordnung die erste interne Aufnahme (7) von der zweiten internen Aufnahme (13) zu trennen:
ein abnehmbarer Sockel (15) ist mit mindestens einem Sensor (16) versehen,
wobei die Teilungswand (14) mit einer Aufnahme (17) versehen ist, die ausgelegt ist, um den abnehmbaren Sockel (15) unterzubringen, der mit zweiten elektrischen Verbindern (11b) versehen ist, die auf lösbare Weise mit den ersten elektrischen Verbindern (10a) gekuppelt werden, wenn der Sockel (15) in der Aufnahme (17) angeordnet ist.

2. Gasdetektorvorrichtung nach Anspruch 1, wobei die Teilungswand (14) eine flache Wand (19) umfasst, die mit einem abgesenkten Abschnitt (21) versehen ist, der die Aufnahme (17) bildet und durch Seitenwände (22) und eine Rückwand (23) begrenzt ist, wobei der abnehmbare Sockel (15) einen Stützabschnitt (24) umfasst, der ausgeformt ist, um in dem abgesenkten Abschnitt (21) untergebracht zu werden und der den Sensor (16) stützt.

3. Gasdetektorvorrichtung nach Anspruch 2, wobei der abgesenkte Abschnitt (21) mit Durchgangsöffnungen (25) versehen ist, die ausgestaltet sind, um den Durchgang der ersten oder zweiten Verbinder (10a, 11b) zu ermöglichen, um die Kupplung zwischen den Verbindern (10a, 11b) zu erzielen.

4. Gasdetektorvorrichtung nach einem der vorhergehenden Ansprüche, wobei die erste Halbschale (6) becherförmig ist und einen im Wesentlichen C-förmigen Querschnitt aufweist, während die zweite Halbschale becherförmig ist und einen im Wesentlichen omegaförmigen Querschnitt aufweist, sodass sie, wenn von der Außenseite betrachtet, einen im Vergleich zu erhöhten seitlichen Abschnitten (M) abgesenkten mittleren Abschnitt (V) aufweist.

5. Gasdetektorvorrichtung nach Anspruch 4, wobei die Öffnungen (4) in dem abgesenkten mittleren Abschnitt und in den seitlichen Abschnitten ausgebildet sind und in einer matrixartigen Struktur angeordnet sind.

6. Gasdetektorvorrichtung nach einem der vorhergehenden Ansprüche, wobei Schnappbefestigungsmittel zwischen den freien Kanten (b1, b2) der ersten becherförmigen Halbschale (6) und der zweiten becherförmigen Halbschale (12) eingesetzt sind.

7. Gasdetektorvorrichtung nach einem der vorhergehenden Ansprüche, wobei Verbindungsmittel zwischen den freien Kanten (b1, b2) der ersten becherförmigen Halbschale (6) und der zweiten becherförmigen Halbschale (12) eingesetzt sind und die Verbindungsmittel Schrauben und/oder einen Klebstoff umfassen.

## Revendications

1. Dispositif détecteur de gaz (1) comprenant une enveloppe de protection (2), au moins un capteur (16) destiné à un type spécifique de gaz logé dans l'enveloppe de protection (2) et un circuit électronique (9) logé dans l'enveloppe (2) et conçu pour détecter un gaz circulant à l'intérieur de l'enveloppe (2) par des orifices d'alimentation en gaz (4), ladite enveloppe comprenant :
i) une première demi-coque (6) définissant un premier logement interne (7) ;
ii) une seconde demi-coque (12), définissant un second logement interne (13), configurée pour s'accoupler avec la première demi-coque (6) et pourvue d'une pluralité d'ouvertures traversantes (4) étant configurées pour établir une communication entre l'extérieur de l'enveloppe (2) et le second logement interne (13) ; et
iii) une cloison de séparation (14),
**caractérisé en ce que** ledit détecteur de gaz comprend en outre : une batterie d'alimentation électrique (8) logée dans ledit premier logement interne (7) ;
une carte principale (9) portant l'électronique de commande du dispositif détecteur, comprenant le microprocesseur (10) et une série de premiers connecteurs électriques (10a) logés dans ledit premier logement interne (7) ;
la cloison de séparation (14) est logée à l'intérieur de ladite enveloppe (2) afin de séparer le premier logement interne (7) du second logement interne (13) en la disposant face aux premiers connecteurs (10a) de la carte principale :
une base amovible (15) pourvue d'au moins un capteur (16) ;
ladite cloison de séparation (14) étant pourvue d'un logement (17) configuré pour loger la base amovible (15) étant pourvue de seconds connecteurs électriques (11b) qui s'accouplent de manière amovible avec les premiers connecteurs électriques (10a) lorsque la base (15) est disposée dans le logement (17).

2. Dispositif détecteur de gaz selon la revendication 1, la cloison de séparation (14) comprenant une paroi plate (19) pourvue d'une partie abaissée (21) qui forme le logement (17) et est délimitée par des parois latérales (22) et une paroi arrière (23) ; la base amovible (15) comprenant une partie de support (24) étant façonnée pour être logée dans la partie abaissée (21) et qui supporte le capteur (16).

3. Dispositif détecteur de gaz selon la revendication 2, la partie abaissée (21) étant pourvue d'ouvertures traversantes (25) conçues pour permettre le passage des premier ou second connecteurs électriques (10a, 11b) afin de réaliser le couplage entre les connecteurs (10a, 11b).

4. Dispositif détecteur de gaz selon l'une quelconque des revendications précédentes, la première demi-coque (6) étant en forme de coupe et présentant une section transversale sensiblement en forme de C, tandis que la seconde demi-coque est en forme de coupe et présente une section transversale sensiblement en forme d'oméga, de sorte qu'elle comporte, vue de l'extérieur, une partie centrale abaissée (V) par rapport à des parties latérales relevées (M).

5. Dispositif détecteur de gaz selon la revendication 4, les ouvertures (4) étant réalisées dans la partie centrale abaissée et dans les parties latérales et étant disposées dans une structure de matrice ordonnée.

6. Dispositif détecteur de gaz selon l'une quelconque des revendications précédentes, des moyens d'encliquetage étant intercalés entre les bords libres (b1, b2) de la première demi-coque (6) en forme de coupe et de la seconde demi-coque (12) en forme de coupe.

7. Dispositif détecteur de gaz selon l'une quelconque des revendications précédentes, des moyens de raccordement étant interposés entre les bords libres (b1, b2) de la première demi-coque (6) en forme de coupe et de la seconde demi-coque (12) en forme de coupe, lesdits moyens de raccordement comprenant des vis et/ou un adhésif.
